# EUROPEAN PATENT SPECIFICATION

(11) **EP 2 595 667 B1**
(45) Date of publication and mention of the grant of the patent: **10.02.2016**
(21) Application number: 11746293.7
(22) Date of filing: 21.07.2011
(51) Int. Cl.: C07K 14/78, A61L 15/32, A61L 27/24, A61L 27/36

(54) **COLLAGENOUS MATERIAL**
KOLLAGENMATERIAL
MATIÈRE COLLAGÈNE

(30) Priority: 22.07.2010 GB 201012307
(43) Date of publication of application: 29.05.2013
(73) Proprietor: Covidien AG, 8212 Neuhausen am Rheinfall (CH)
(72) Inventor: ARMITAGE, Paul, P015 7NY Fareham (GB); DAWSON, Christine Elizabeth, P015 7NY Fareham (GB)
(74) Representative: Soames, Candida Jane
(86) International application number: PCT/GB2011/051389
(87) International publication number: WO 2012/010903

(56) References cited:
- WO-A1-00/15274
- WO-A1-00/29484
- WO-A1-85/05274
- WO-A2-2008/125850
- WO-A2-2008/125851
- US-A- 4 412 947
- US-A- 4 953 299
- US-A- 5 331 092
- SCHOOF H ET AL: "Control of pore structure and size in freeze-dried collagen sponges.", JOURNAL OF BIOMEDICAL MATERIALS RESEARCH 2001 LNKD- PUBMED:11410892, vol. 58, no. 4, 2001, pages 352-357, XP002662523, ISSN: 0021-9304

## Description

The present invention relates to a collagenous material and to a process for the manufacture thereof. The collagenous material may be used, for example, in wound care.

Collagen is a triple-helix protein which forms the major part of the dermal extracellular matrix (ECM), together with glycosaminoglycans, proteoglycans, laminin, fibronectin, elastin and cellular components. The ECM is the largest component of the dermal skin layer and the synthesis of ECM is a key feature of wound healing, especially where there has been a significant loss of tissue that precludes closure by primary intention.

The principal function of collagen in the dermal ECM is to act as a scaffold in connective tissue. Predominantly, collagen is present in the form of type I collagen (80-85%) and type III collagen (8-11%), both of which are fibrillar or rod-shaped collagens. The tensile strength of skin is due largely to these collagen molecules assembling into fibrils, with adjacent molecules crosslinking to further increase tensile strength. However, collagen laid down during wound healing has a reduced structural integrity compared to unwounded tissue; scar tissue rarely exceeds 70% of unwounded tissue strength.

In addition to being the main component of scar tissue, collagen has a key role in the control of the inflammatory response to injury and subsequent repair, with functions that influence cellular mitogenesis, differentiation and migration; protein synthesis in the ECM; synthesis and release of inflammatory cytokines and growth factors; and interactions between enzymes which remodel the ECM, including matrix metalloproteinases (MMPs) and their tissue inhibitors (TIMPs).

For medical applications, purified fibrous collagen has long been known to be a useful therapeutic adjunct to aid wound healing. Research into collagen-based dressing materials has shown that collagen significantly increases the production of fibroblasts in wounds, encouraging direct migration of cells into the wound, enhancing the formation of new, organised collagen fibres.

Purified fibrous collagen pads have also been used to provide haemostatic dressings. When in contact with a bleeding surface, a fibrous collagen haemostat attracts platelets which adhere to collagen fibrils and release coagulation factors. This coagulation and aggregation of the platelets into thrombi on the collagenous mass, provides the formation of a physiologic platelet plug which slows and eventually stops the bleeding. The collagen fibrils also provide a structural matrix strengthening the platelet plug.

Previous studies have demonstrated that collagenous materials prepared using a process that retains the original fibre architecture and molecular ultrastructure of the natural tissues from which they are derived, have a number of advantageous properties. For example, US 5397353 discloses a process for the preparation of collagenous materials that are substantially non-antigenic and substantially free of non-fibrous tissue proteins, cellular elements, lipids and lipid residues. The materials are typically sheet structures and are useful, for example, as implants. The collagenous sheet materials are susceptible to colonisation and vascularisation by host cells following implantation, and are resistant to calcification.

It has further been shown that the favourable properties of these collagen sheet materials can be retained when the collagen material is presented in the form of particles comprising fibre fragments. EP 1112096 describes an injectable or mouldable composition of collagen fibre fragments prepared from the relatively large-scale sheet materials of US 5397353 in such a way as to retain the natural collagen fibre architecture and molecular ultrastructure. This size reduction is achieved by careful grinding or milling of the collagenous sheet materials, which may then be suspended to form a paste or injectable composition, as appropriate.

The present invention provides a new collagenous material in accordance with the claims particularly suitable for use as a wound dressing.

According to an aspect of the present invention there is provided a process defined by Claim 1 for the manufacture of a coherent collagenous material.

Freeze drying the collagen particles gives rise to a coherent collagenous material. Surprisingly, the freeze-drying step does not result in a dry powder of collagen particles as would be expected. Unlike typical existing processes for manufacturing collagenous materials, the present invention does not require harsh chemical or mechanical treatment of the collagen starting material. The collagen particles themselves are initially prepared in such a way as to preserve original collagen fibre architecture and molecular ultrastructure of the natural tissue material from which they are derived, care being taken to avoid chemical or mechanical damage. Moreover, further processing of the collagen particles in the present invention avoids chemical or mechanical treatments that can damage the original collagen fibre architecture and molecular ultrastructure of the collagen fibre fragments making up the collagen particles. Thus, the collagenous material of the present invention comprises collagen displaying original collagen fibre architecture and molecular ultrastructure of the natural tissue material from which the collagen is derived.

In particular, in contrast to the known processes, the present invention does not require treatment of the collagen particles with acid to swell or solubilise the collagen. This is a significant advantage, since acid treatment is known to have a detrimental effect on the structure and integrity of the collagen fibres. Thus, the present invention excludes treatment of the collagen particles with acid, in particular swelling of the collagen particles with acid.

Similarly, many prior art processes involve alkali treatment of collagen which is also known to be damaging to collagen. Thus, the present invention excludes treatment of the collagen particles with alkali, in particular swelling of the collagen particles with alkali.

Thus, damage by acid or alkali swelling of collagen is avoided. Conditions for swelling collagen with acid and alkali are known in the art. As a guide, to minimise the risk of damage to the structure of the collagen, cross-linked collagen particles should preferably be maintained within a pH range of around 5 to 12, whereas non-cross-linked collagen particles should preferably be maintained within a pH range of about 6 to 8.

The present invention also avoids the need for extensive homogenisation associated with many existing processes, which can lead to mechanical damage to the collagen fibres. Thus, preferred embodiments of the present invention exclude homogenisation step(s).

The collagenous material has good properties for use as a wound dressing or as a component of a wound dressing. This porous, biocompatible material is non-antigenic and of natural origin, and provides a soft, sponge-like feel. The collagenous material is flexible, with a degree of conformability, which is clearly advantageous for application to wound sites. Significantly, the collagenous material has good cohesive strength and may be wrapped around wound areas to form an effective barrier. The collagenous material maintains a moist wound environment to promote wound healing, and its application helps to reduce contraction and scar tissue formation.

Being formed from a plurality of collagen particles, the collagenous material has a relatively uniform and consistent structure.

Further, the presence of collagen at a wound site is known to increase the rate of wound healing.

Thus, the collagenous material described herein is particularly useful in wound care. The collagenous material may be presented for use in a hydrated state. The process may therefore optionally further comprise at least one rehydration step, in which the collagenous material is maintained in an aqueous solution. For instance, the collagenous material may be maintained in saline, such as 0.9% saline.

The presentation of the collagenous material in a hydrated form helps to maintain a moist wound environment when the material is applied as a wound dressing. Unexpectedly, it has been found that the collagenous material formed from collagen particles remains intact following processing/manufacture and does not return to a particulate state following rehydration. Further, the collagenous material does not gel upon rehydration as seen with certain other wound dressings.

The coherent collagenous material produced using the process of the present invention may be manufactured in essentially any size, and can therefore be used to provide coverage of large wound sites, such as bums. The structural integrity of the collagenous material provides good protection to a wound site and the collagenous material may be readily secured in position to prevent migration.

The skilled person is aware that 'freeze drying', also referred to as 'lyophilisation', involves drying a material in a frozen state at very low pressure (i.e. in a high vacuum) so that ice, or other frozen solvent, sublimes rapidly without melting. Methods and apparatus for freeze drying are well known in the art (see, e.g., 'Freeze-Drying/Lyophilization Of Pharmaceutical and Biological Products', Third Edition, Louis Rey and Joan C. May, Informa Healthcare, 2010). The step of freeze drying may be carried out by any suitable method and using any suitable apparatus.

Any suitable freeze-drying suspension medium may be employed. For example, the freeze-drying suspension medium may comprise water or an aqueous medium, such as saline. The invention excludes the use of freeze-drying suspension media containing collagen-swelling levels of acids or alkalis, in order to avoid damage to the fibre architecture and molecular ultrastructure of the collagen particles. By way of example, freeze-drying suspension media having a pH within the range of about 5 to 12 may be used to suspend cross-linked collagen particles, whereas for non-cross-linked collagen particles the freeze-drying suspension media may have a pH within the range of about 6 to 8.

The collagen particles may be suspended in the freeze-drying suspension medium at a range of different concentrations. Typically, the concentration of collagen particles suspended in the freeze-drying suspension medium is in the range of 10 to 80% (w/v), although other concentrations may be used. At the higher end of this range, the suspension of collagen particles in the freeze-drying medium has a relatively thick, pasty consistency.

The process may optionally include a step of mixing the suspension of collagen particles in the freeze-drying suspension medium. This ensures even distribution of the collagen particles in the freeze-drying suspension medium. Harsh mechanical mixing treatments such as homogenisation or blending are preferably avoided, however, in order to minimise collagen damage.

The natural tissue material may comprise any collagen-containing tissue material of human or animal (i.e. non-human) origin. The natural tissue material may be a tissue comprising predominantly type I collagen. Preferred starting materials include dermis and tendons. In some embodiments, it is preferred that porcine tissue materials are processed to provide the collagenous material, although it will be understood that other mammalian sources may alternatively be employed, such as, for example, primates, cows, sheep, goats or horses.

Depending upon the starting material, the particles of collagen may contain a proportion of elastin. Thus, the particles, and the coherent collagenous materials formed therefrom, consist essentially of collagen optionally with small proportions of elastin.

The particles of acellular collagen may be formed using any suitable process. The original collagen fibre architecture and molecular ultrastructure of the natural tissue material must be retained in the particles. Preferred processes for preparing the collagen particles are therefore analogous to those described in EP 1112096. Preferably, collagenous material prepared in the form of a sheet or other large-scale structure is milled to form the particles. The collagenous material may be prepared by a process analogous to those described in US 5397353. The collagenous tissue is neither solubilised nor denatured in the process, so its natural fibre structure is maintained.

Thus, freshly cut natural tissue material is treated to remove therefrom substantially all lipids and lipid residues and thereafter treated to remove non-fibrous tissue proteins and cellular elements.

The lipid extraction may be achieved by solvent extraction using an orgamc solvent, such as acetone. Other non-limiting examples of suitable solvents include nonaqueous solvents such as ethanol and ether.

Non-fibrous tissue proteins include glycoproteins, proteoglycans, globular proteins and the like, such as components of extracellular matrix. Cellular elements include antigenic proteins and enzymes and other cellular debris arising from the processing conditions. These portions of the natural tissue material may be removed by treatment with a proteolytic enzyme, such as trypsin. It has previously been found that above 20°C treatment with trypsin can in some circumstances result in an alteration of the collagen fibre structure leading to a lower physical strength. Moreover, low temperatures discourage the growth of microorganisms in the preparation. It is therefore preferred to carry out the treatment with trypsin at a temperature below 20°C. Moreover, trypsin is more stable below 20°C and lower amounts of it may be required. Any suitable trypsin concentration may be used, for instance a concentration within the range of around 0.01g/l to 25g/l. It has been found that good results can be obtained using about 2.5g/l trypsin.

Further treatments may optionally be carried out, such as treatment with one or more additional enzymes, for example a carbohydrate-splitting enzyme.

Those substances said to be "substantially free" of materials generally contain less than 5% of, and preferably less than 1%, of said materials.

The resulting collagenous material is then reduced to particles, care being taken to ensure that the size reduction is not associated with a degradation of the original collagen fibre architecture and molecular ultrastructure of the starting material. The particles are produced by grinding or milling using, for example, a ball or hammer mill, which may be cooled to an appropriate temperature. The sheet material may be cut into small pieces prior to milling. Milling may be carried out in dry form (less than 10% moisture content)
or in frozen hydrated form (20-80% moisture content).

The collagen particles may be of any suitable slze. Typically, the collagen particles have a mean diameter within the range of from around 5flm to around lOOOflm, more typically from around 50flm to around 500flm. Good results have been achieved using collagen particles with a mean diameter of approximately 150flm.

The freeze-dried collagenous material is porous, with a sponge-like appearance.

The process may optionally include a step of shaping or moulding the suspension of collagen particles to be freeze dried. Usefully, this allows the shape or conformation of the resulting freeze-dried collagenous material to be varied accordingly.

The suspension of collagen particles may, for example, be moulded, formed or cast into the desired shape prior to freeze-drying. Typically, the freeze-drying suspension medium and collagen particles suspended therein are held in a suitable mould during the freeze-drying step. The process can therefore be used for the manufacture of collagenous materials of predetermined shapes, which can readily be varied by using different moulds.

Non-limiting examples of suitable shapes include sheets, plugs, blocks, wedges, beads, ropes, or variations thereof.

Optionally, cross-linking may be carried out to impart additional physical strength to the collagenous material, and an increased resistance to digestive enzymes that may be present in a wound healing environment. Surprisingly, freeze-dried collagenous material formed from particles of cross-linked collagen has been found to have improved flexibility as compared to corresponding collagenous material formed from non-cross-linked collagen particles. This is beneficial for use of the collagenous material in wound care. Thus, preferred embodiments of the present invention include a step of cross-linking the collagen particles.

Whilst any cross-linking agent may be used, preferred cross-linking agents include polyisocyanates, in particular diisocyanates. The polyfunctional isocyanates react with amino or hydroxyl groups of different protein chains so forming a material which has a stable structure retaining the original architecture of the collagen and which is resistant to enzymatic attack. It is known that antigenicity is associated with the amino groups of the protein chains of collagen, and reacting the amino groups with isocyanate removes any antigenicity associated with these groups. Preferred diisocyantes include aliphatic, aromatic and alicyclic diisocyanates as exemplified by 1,6-hexamethylene diisocyanate, toluene diisocyanate, 4,4'-diphenylmethane diisocyanate, and 4,4'-dicyclohexylmethane diisocyanate, respectively. A particularly preferred diisocyanate is hexamethylene diisocyanate (HMDI). Carbodiimide cross-linking agents may also be used, such as 1-ethyl-3-[3-dimethylaminopropyl]carbodiimide hydrochloride (EDC).

The extent of cross-linking of the collagen particles may be varied. Usefully, this provides a mechanism for controlling the rate at which the collagenous material is resorbed or degraded during use. The resistance to degradation tends to increase as the extent of cross-linking is increased.

By way of example, cross-linking of the collagen particles may be carried out using HMDI. As a guide, the HMDI may be used at a concentration of around 0.01g to 0.5g per 50g of collagen. If the concentration is too high, this may result in over-cross-linking and foreign body reactions. Cross-linking may be carried out for a range of different time periods. By way of example, the collagen may be exposed to the cross-linking agent for between around 1 hour and around 3 days. Typically, cross-linking is carried out for at least 12 hours, preferably at least 20 hours.

It will be appreciated that the cross-linking conditions may routinely be varied in order to adjust the extent of cross-linking.

According to a further aspect of the present invention there is provided a coherent collagenous material obtainable by a process as herein described.

A range of different factors may be added to the collagenous material, such as growth factors, clotting agents, or other pharmaceutically active agents. The collagenous material may be seeded with cells, such as stem cells.

The collagenous material is useful in wound care. The collagenous material may also be used as a haemostat, to reduce or prevent blood flow from a body site.

According to a further aspect of the present invention there is provided a collagenous material as herein described for use in therapy.

According to a further aspect of the present invention there is provided a collagenous material as herein described for use in wound care.

According to a further aspect of the present invention there 1s provided a collagenous material as herein described for use as a haemostat.

Embodiments of the present invention will now be described further in the following non-limiting examples.

### Examples

### 1. Manufacture of freeze-dried collagenous material

Acellular collagen sheet material was prepared according to the method disclosed in US 5397353 and reduced to particles as described in EP 1112096.

Thus, freshly cut dermis harvested from sows was immersed in acetone. After 1 hour, the acetone was removed and replaced by fresh acetone. After a further incubation for around 36 hours, the tissue was removed from the acetone and placed in 0.9% saline to extract residual acetone. The tissue was then digested for 28 days with a solution of trypsin at a concentration of 2.5 mg/ml in O.lM phosphate buffer with 0.5 mg/ml sodium azide as a bacteriostatic agent. The purified tissue was removed from the trypsin solution and rinsed in buffer.

The resulting collagenous sheet material was cut into small pieces (approximately 5 x 10 mm) before being milled cryogenically to a mean particle size of around 150µm.

The collagen particles were then cross-linked using HMDI in acetone. A concentration of about 0.1g HMDI per 25g of solid collagen was added. The collagen was cross-linked for at least 20 hours and rinsed in acetone.

The cross-linked collagen particles were suspended in a freeze-drying suspension medium, being 0.9% saline. The suspension of collagen particles was placed in a polypropylene mould and freeze dried.

Freeze drying was carried out in a VirTis Advantage Plus EL freeze-dryer (vacuum set point 600 mTorr; condenser set point -45°C), using the freeze-drying cycle shown below.

Thermal treatment steps:

| Time (mins.) | Temp. (°C) | Hold/Ramp |
|---|---|---|
| 1 | 20 | H |
| 110 | -35 | R |
| 70 | -35 | H |

Drying steps:

| Time (mins) | Temp. (°C) | Hold/Ramp |
|---|---|---|
| 5 | -35 | H |
| 30 | -20 | R |
| 330 | -20 | H |
| 80 | 20 | R |
| 340 | 20 | H |

The resulting collagenous material was a flexible, sponge-like sheet having the general shape of the polypropylene mould. The sheet was porous, with a soft texture and had good physical integrity.

When rehydrated, the absorbent collagenous material quickly swelled. The collagenous material did not gel or fragment, and had good cohesive structural integrity.

### 2. Effect of cross-linking of the collagen particles

The process of Example 1 was repeated but the step of cross-linking the collagen particles with HMDI was omitted.

As with the collagenous material produced from cross-linked collagen particles, the collagenous material formed by freeze-drying non-cross-linked collagen particles also retained the general shape of the polypropylene mould. When rehydrated, neither material gelled or fragmented or returned to particulate form.

The collagenous material formed from cross-linked collagen particles was found to be softer and more flexible than the collagenous material formed from non-cross-linked collagen particles. Upon rehydration with saline, the former absorbed moisture more quickly and became more swollen. In rehydrated form, the collagenous material formed from cross-linked collagen particles had a sponge-like texture, whereas the collagenous material formed from non-cross-linked collagen particles was smoother and more dense.

It is of course to be understood that the invention is not intended to be restricted by the details of the above specific embodiments, which are provided by way of example only.

## Claims

1. A process for the manufacture of a coherent collagenous material from a natural tissue material, wherein said process comprises steps of:
(i) treating the natural tissue material with an organic solvent; and then
(ii) treating the natural tissue material with a proteolytic enzyme,
so as to provide a treated material that is free of non-fibrous tissue proteins, cellular elements and lipids or lipid residues and displays original collagen fibre architecture and molecular ultrastructure of the natural tissue material from which it is derived; and then
(iii) reducing said treated material by milling or grinding to a plurality of collagen particles having a mean diameter within the range of 5 µm to 1000µm comprising fragments of collagen fibres; and subsequently
(iv) freeze drying said collagen particles in suspension in a freeze-drying suspension medium, wherein said process excludes swelling of the collagen particles with acid and/or alkali.

2. A process according to claim 1, wherein the process excludes homogenisation of the collagen particles.

3. A process according to claim 1, wherein the process includes a step of cross-linking the collagen particles.

4. A process according to claim 1, wherein the process includes a step of shaping the suspension of collagen particles in the freeze-drying suspension medium.

5. A process according to claim 1, wherein the suspension of collagen particles in the freeze-drying suspension medium is held in a mould for the step of freeze drying.

6. A process according to any one of the preceding claims, wherein the process includes a step of rehydrating the collagenous material.

7. A process according to claim 1, wherein the proteolytic enzyme comprises trypsin.

8. A process according to any one of the preceding claims, wherein the natural tissue material is of non-human origin.

9. A coherent collagenous material which is free from non-fibrous tissue proteins, cellular elements and lipids or lipid residues and displays original collagen fibre architecture and molecular ultrastructure of the natural tissue material from which it is derived, and is formed of a plurality of milled or ground collagen particles suspended in a freeze-drying suspension medium in accordance with the process of any one of the preceding claims.

10. A collagenous material according to claim 9 for use in therapy.

11. A collagenous material according to claim 9 or claim 10 for use in wound care.

12. A collagenous material according to claim 9 or claim 10 for use as a haemostat.

## Patentansprüche

1. Ein Verfahren zur Herstellung eines kohärenten Kollagenmaterials aus natürlichem Gewebe, wobei dieses Verfahrens die folgenden Schritte umfasst:
(i) Behandlung des natürlichen Gewebematerials mit einem organischen Lösungsmittel; und dann
(ii) Behandlung des natürlichen Gewebematerials mit einem proteolytischen Enzym,
um so ein behandeltes Material bereitzustellen, dass frei von nicht faserhaltigen Gewebeproteinen, Zellelementen und Lipiden oder Lipidrückständen ist und eine Originalkollagenfaserstruktur und eine molekulare Ultrastruktur des natürlichen Gewebematerials, von dem es abgeleitet wird, aufweist; und dann
(iii) Reduzieren dieses behandelten Materials durch Mahlen und Reiben in eine Vielzahl von Kollagenteilchen mit einem mittleren Durchmesser innerhalb des Bereichs von 5 µm bis 1.000 µm, darunter Fragmente von Kollagenfasern; und danach
(iv) Gefriertrocknen der genannten Kollagenteilchen in Suspension, in einem Gefriertrocknungssuspensionsmedium, wobei dieses Verfahren das Quellen der Kollagenteilchen mit Säure und/oder Basen ausschließt.

2. Ein Verfahren gemäß Anspruch 1, wobei das Verfahren eine Homogenisierung der Kollagenteilchen ausschließt.

3. Ein Verfahren gemäß Anspruch 1, wobei das Verfahren einen Schritt der Vernetzung der Kollagenteilchen einschließt.

4. Ein Verfahren gemäß Anspruch 1, wobei das Verfahren einen Schritt der Ausformung der Kollagenteilchensuspension im Gefriertrocknungssuspensionsmedium einschließt.

5. Ein Verfahren gemäß Anspruch 1, wobei die Kollagenteilchensuspension im Gefriertrocknungssuspensionsmedium für den Schritt des Gefriertrocknens in einer Schablone gehalten wird.

6. Ein Verfahren gemäß einem der vorhergehenden Ansprüche, wobei das Verfahren einen Schritt der Rehydrierung des Kollagenmaterials einschließt.

7. Ein Verfahren gemäß Anspruch 1, wobei das proteolytische Enzym Trypsin umfasst.

8. Ein Verfahren gemäß einem der vorhergehenden Ansprüche, wobei das natürliche Gewebematerial nicht menschlicher Herkunft ist.

9. Ein kohärentes Kollagenmaterial, das frei von nicht faserhaltigen Gewebeproteinen, Zellelementen und Lipiden oder Lipidrückstellungen ist und eine Originalkollagenfaserstruktur und eine molekulare Ultrastruktur des natürlichen Gewebematerials, von dem es abgeleitet wird, aufweist; und das aus einer Vielzahl zermahlener und zerriebener Kollagenteilchen, die in einem Gefriertrocknungssuspensionsmedium suspendiert sind, in Übereinstimmung mit dem Verfahren eines der vorhergehenden Ansprüche gebildet wird.

10. Ein Kollagenmaterial gemäß Anspruch 9 zum Einsatz in der Therapie.

11. Ein Kollagenmaterial gemäß Anspruch 9 oder Anspruch 10 zum Einsatz in der Wundbehandlung.

12. Ein Kollagenmaterial gemäß Anspruch 9 oder Anspruch 10 zum Einsatz als Haemostatika.

## Revendications

1. Processus pour la fabrication d'une matière collagène cohérente à partir d'une matière tissulaire naturelle, dans lequel ledit processus comprend les étapes de :
(i) traitement de la matière tissulaire naturelle avec un solvant organique ; et ensuite
(ii) traitement de la matière tissulaire naturelle avec une enzyme protéolytique,
de façon à fournir une matière traitée qui est exempte de protéines tissulaires non fibreuses, d'éléments cellulaires et de lipides ou de résidus de lipide, et qui affiche une architecture de fibre de collagène originale et une ultrastructure moléculaire de la matière tissulaire naturelle à partir de laquelle elle est obtenue ; et ensuite
(iii) réduction de ladite matière traitée par broyage ou concassage en une pluralité de particules de collagène ayant un diamètre moyen dans la plage de 5 µm à 1 000 µm comprenant des fragments de fibres de collagène ; et par la suite
(iv) lyophilisation desdites particules de collagène en suspension dans un milieu de suspension pour lyophilisation, dans lequel ledit processus exclut le gonflement des particules de collagène avec un acide et/ou un alcali.

2. Processus selon la revendication 1, dans lequel le processus exclut l'homogénéisation des particules de collagène.

3. Processus selon la revendication 1, dans lequel le processus inclut une étape de réticulation des particules de collagène.

4. Processus selon la revendication 1, dans lequel le processus inclut une étape de mise en forme de la suspension de particules de collagène dans le milieu de suspension pour lyophilisation.

5. Processus selon la revendication 1, dans lequel la suspension de particules de collagène dans le milieu de suspension de lyophilisation est maintenue dans un moule pour l'étape de lyophilisation.

6. Processus selon n'importe laquelle des revendications précédentes, dans lequel le processus inclut une étape de réhydratation de la matière collagène.

7. Processus selon la revendication 1, dans lequel l'enzyme protéolytique comprend de la trypsine.

8. Processus selon n'importe laquelle des revendications précédentes, dans lequel la matière tissulaire naturelle est d'origine non humaine.

9. Matière collagène cohérente qui est exempte de protéines tissulaires non fibreuses, d'éléments cellulaires et de lipides ou de résidus de lipide, et qui affiche une architecture de fibre de collagène originale et une ultrastructure moléculaire de la matière tissulaire naturelle à partir de laquelle elle est obtenue, et est formée d'une pluralité de particules de collagène broyées ou moulues suspendues dans un milieu de suspension pour lyophilisation selon le processus de n'importe laquelle des revendications précédentes.

10. Matière collagène selon la revendication 9 pour utilisation dans une thérapie.

11. Matière collagène selon la revendication 9 ou la revendication 10 pour utilisation dans des soins de blessure.

12. Matière collagène selon la revendication 9 ou la revendication 10 pour utilisation en tant qu'hémostat.
